# EUROPEAN PATENT SPECIFICATION

(11) **EP 4 461 492 B1**
(45) Date of publication and mention of the grant of the patent: **08.04.2026**
(21) Application number: 24202325.7
(22) Date of filing: 06.12.2023
(51) Int. Cl.: C08G 12/12, C09J 161/24, B27N 1/00, B27N 1/02, B27N 3/00, B27N 3/02, B27N 3/04, B27N 3/18, B27N 7/00, B27N 9/00, B27N 3/24

(54) **METHOD FOR PRODUCING A UREA-FORMALDEHYDE GLUE**
VERFAHREN ZUR HERSTELLUNG EINES HARNSTOFF-FORMALDEHYD-LEIMS
PROCÉDÉ DE FABRICATION D'UNE COLLE URÉE-FORMALDÉHYDE

(30) Priority: 16.12.2022 BE 202206029
(43) Date of publication of application: 13.11.2024
(62) Divisional of application: 23214712.4
(73) Proprietor: Unilin, BV, 8710 Wielsbeke (BE)
(72) Inventor: Melis, Mathilde, 8710 Wielsbeke (BE); Bilcke, Wannes, 8710 Wielsbeke (BE); Lambregts, Martijn, 8710 Wielsbeke (BE); Van Hezel, Sven, 8710 Wielsbeke (BE); Baekelandt, Bert, 8710 Wielsbeke (BE)
(74) Representative: Unilin Technologies

(56) References cited:
- WO-A1-89/00587
- US-A1- 2002 128 398
- US-B1- 6 414 312

## Description

The present invention relates to a method for producing a urea-formaldehyde glue. Said urea-formaldehyde glue can be used for boards comprising a wood-based substrate, wherein this wood-based substrate comprises wood particles and said urea-formaldehyde glue which binds these wood particles together, wherein for producing the substrate:
- said urea-formaldehyde glue is produced;
- wood particles are produced;
- the wood particles are coated with at least the urea-formaldehyde glue, after which the glue-coated wood particles are pressed to form a plate shape;
and that, in order to manufacture the urea-formaldehyde glue, raw materials of this glue are combined in a reactor, wherein these raw materials at least comprise urea, water and a urea-formaldehyde concentrate, wherein the urea-formaldehyde concentrate is produced by combining urea, formaldehyde and water, and the resultant urea-formaldehyde concentrate is introduced into a storage tank with the aid of a first line, wherein the urea-formaldehyde concentrate is introduced into the reactor from the storage tank with the aid of a second line.

These boards may, for example, be rough or sanded boards, wherein the wood particles may comprise wood fibres, wood chips or wood shavings which may or may not come from recycling wood waste. These boards are then, for example, wood fibreboard, such as MDF - Medium Density Fibreboard - and HDF - High Density Fibreboard -, or these boards are particleboard or OSB (Oriented Strand Board). These boards may or may not be partly or entirely made of recycled material. These recycled materials originate, for example, from recycling particleboard, wood fibreboard or other boards, such as panels comprising such particleboard and wood fibreboard. Thus, particleboards may comprise particles of recycled particleboards or wood fibreboards may comprise particles of recycled wood fibreboards. The abovementioned substrate in this case forms the board as such.

These boards may also be decorative boards and may then be referred to, for example, as decorative panels. In that case, these decorative panels are, for example, floor panels, wall panels, furniture panels, etc. These decorative panels then comprise the abovementioned substrate - such as an MDF, an HDF or a particleboard board - and a decorative top layer and optionally a balance layer which extends on the other side of the substrate. This decorative top layer may then consist of one or more layers, wherein these layers may comprise: a resin-impregnated paper - such as a melamine resin-impregnated paper -, a lacquer layer, a varnish layer, a glue layer, a web - such as a woven, a nonwoven, a glass fiber web -, a veneer, etc.

These boards may have a specific function, such as fire resistance and/or electrical conductivity and/or water repellency, in which case, for example, a fire-resistant and/or electrically conductive and/or water-repellent layer is then applied to the substrate. In addition, fire-resistant and/or electrically conductive and/or water-repellent components may be present in the substrate. These boards may optionally be provided with a decorative layer.

Here, the term urea-formaldehyde glue is generally understood to refer to a glue which is formed from at least urea and formaldehyde, for example produced via a polycondensation reaction of urea and formaldehyde. Optionally, it is also possible to add melamine in order to form a melamine urea-formaldehyde glue (MUF glue). Optionally, melamine and phenol may be added in order to form a melamine urea-formaldehyde phenol glue (MUPF glue). If no melamine or other additives are added, the resultant product is referred to as a UF glue. In this case, a urea-formaldehyde glue may refer to a UF glue and a MUF glue as well as a MUPF glue.

When forming these boards and also with the resultant boards, the ratio formaldehyde/urea of the urea-formaldehyde glue which is used to form the substrate is an important parameter. If this ratio is too low, this may result in problems in the production, for example compressing the substrate may proceed less quickly or the strength of the resultant substrate may be insufficient, but also any production steps which take place after the substrate has been produced may suffer from problems, for example the adhesion of a said top layer and/or a balance layer to the substrate may be insufficient and/or be slower, the installation of coupling parts at the level of the substrate may be less accurate, etc. If the abovementioned ratio is too high, this may result in high formaldehyde emissions. Thus, the formaldehyde emission may be higher than standards allow. The properties of the urea-formaldehyde glue used are then also crucial for the quality and the uniformity of the resultant substrate.

The wood particles themselves may also already have a certain formaldehyde content. In particular, when wood particles originating from wood waste, such as wood waste comprising particleboard, wood fibreboard and the like, are used, these wood particles may have a high formaldehyde content, as such wood waste usually comprises urea-formaldehyde glue. As the composition of the wood waste may vary greatly, the uniformity of the resultant boards is low when use is made of non-uniform wood waste. The term wood particles here not only refers to particles which comprise solely wood components. They may, for example, also comprise glues and/or contaminants. US 2002128398 A1 discloses the preamble of claim 1.

This application discloses a method for producing boards comprising a wood-based substrate, wherein this wood-based substrate comprises wood particles and a urea-formaldehyde glue which binds these wood particles together, wherein for producing the substrate:
- a urea-formaldehyde glue is produced;
- wood particles are produced;
- the wood particles are coated with at least the urea-formaldehyde glue, after which the glue-coated wood particles are pressed to form a plate shape;
and that, in order to produce the urea-formaldehyde glue, raw materials of this glue are combined in a reactor, wherein these raw materials at least comprise urea, water and a urea-formaldehyde concentrate (UFC), wherein the UFC is produced by combining urea, formaldehyde and water, and the resultant UFC is introduced into a storage tank with the aid of a first line, wherein the urea-formaldehyde concentrate is introduced into the reactor from the storage tank with the aid of a second line, wherein formaldehyde/urea (F/U) molar ratios of the UFC are determined on the basis of NIR measurements and at least one calibration model, and that these NIR measurements are performed on UFC in at least two locations, being a first location which is situated in the first line and a second location which is situated in the storage tank or the second line, and wherein the raw materials are dosed into the reactor on the basis of said F/U molar ratios of the UFC.

Here, the term NIR measurements is used to denote measurements which are performed near the infrared part of the electromagnetic spectrum (near-infrared measurements, infrared spectroscopy). With the aid of NIR measurements, non-destructive measurements may be performed on the UFC.

UFC is a reaction product of formaldehyde and urea, and has a composition of, for example: 23 to 25% by weight of free (unreacted) formaldehyde, 55 to 57% by weight of oligomers and polymers of urea and formaldehyde, and 19 to 21% by weight of water.

The raw material urea which is dosed into the reactor may be pure urea, but may also comprise other constituents. An example of a possible raw material urea is the following: 98.4 to 98.9% by weight of urea (NH₂-CO-NH₂), 0.2 to 0.4% by weight of water, 0.2 to 0.3% by weight of UFC, 0.7 to 0.9% by weight of biuret, traces of ammonium salts, such as cyanate and carbamate.

In order to produce UFC, urea is preferably dissolved in water, and this water comprising urea is brought into contact with formaldehyde gas. This water comprising urea comprises, for example, between 56 and 58% by weight of urea.

NIR measurements and a calibration model are useful in order to determine the F/U molar ratio of the UFC. However, it was found that with NIR measurements of the UFC of the storage tank, the accuracy of the determined F/U molar ratio was insufficient and consequently the quality of the urea-formaldehyde glue produced often left something to be desired. Surprisingly, it was found that by measuring in at least two locations, namely at least on UFC of said first location and the second location, the accuracy increases significantly, also resulting in a more uniform quality of the resultant urea-formaldehyde glue and, in addition, the urea-formaldehyde glue can be produced more quickly, as possible corrections to the urea-formaldehyde glue are no longer necessary and/or fewer corrections are required. Due to the improved quality of the resultant urea-formaldehyde glue, the production process of boards may be accelerated and/or the quality of the resultant boards is more uniform and/or fewer corrective measures to compensate for the inadequate quality of the urea-formaldehyde glue are required. The NIR measurements at the first location mainly assist in optimizing the UFC production, as any variations can quickly be detected and thus it is possible to rapidly intervene during the UFC production. The NIR measurements at the second location are very useful when dosing UFC during glue production.

NIR measurements and a calibration model are useful when determining the F/U molar ratio of the UFC. Preferably, this calibration model is used to determine the carbon and nitrogen content and furthermore the NIR measurements and the corresponding calibration model are preferably used to determine not only the F/U molar ratio, but also the formaldehyde content, the urea content and the water content. In this way, the entire composition of the UFC is known. Where this text mentions that F/U molar ratios of the UFC are determined on the basis of NIR measurements and at least one calibration model, this therefore preferably means that F/U molar ratios, formaldehyde content, urea content and water content of the UFC are determined on the basis of NIR measurements and at least one calibration model. When dosing takes place and/or modifications are carried out on the basis of F/U molar ratios of the UFC, it is preferably indicated that dosing is performed and/or modifications are carried out on the basis of F/U molar ratios, formaldehyde content, urea content and water content of the UFC. If the F/U molar ratio of UFC is mentioned, the following is thus preferably mentioned: F/U molar ratio, formaldehyde content, urea content and water content of the UFC.

The NIR measurements of the UFC at said two locations may be performed online and/or offline.

The term online NIR measurements (synonym inline NIR measurements) denotes NIR measurements of the UFC at that location itself. This may be done, for example, by placing a probe/sensor at the said location, for example by placing a probe/sensor in a said line or in the storage tank. The advantage of online NIR measurements is that these measurements are performed in situ, as a result of which they can be carried out more quickly. This also allows for quick interventions, for example a quick intervention during UFC production, as a result of which the uniformity of the produced UFC can be very high. Also, there is no need to take samples, no need to prepare samples and also no need to transport the sample to, for example, a laboratory. There is then also a smaller risk of human error and thus faulty measurements. Moreover, it is possible to use continuous NIR measurements here, as a result of which it is possible, if required and on the basis of determined F/U molar ratios, to intervene quickly in the production of the UFC and/or to intervene quickly in the dosing of the raw materials into the reactor when producing the urea-formaldehyde glue. In this case, recent measurements and consequently correct values are used when dosing the raw materials, as a result of which dosing can be performed accurately.

The term offline NIR measurements denotes NIR measurements using one or more NIR measuring apparatuses which are arranged at a distance from said locations. These measuring apparatuses may be arranged in the production environment of the UFC and/or the glue, but may also be arranged in a separate laboratory. By working offline, the environmental parameters, such as for example temperature, atmospheric humidity, flow velocity, etc., can be controlled more securely, making it possible to produce very accurate measurements. Preferably, a sample is taken at said locations and then NIR measurements are performed on it.

Preferably, the calibration models used are optimized continuously or at specific points in time with the aid of, for example, reference samples and/or NIR measurements of the UFC performed at said locations and/or corresponding wet-chemical measurements of the UFC at said locations. Wet chemistry, or general chemistry, describes a wide range of classical chemical analyses. The formaldehyde content, urea content, water content and F/U molar ratio of the UFC are therefore determined with the aid of classical chemical analyses, with the corresponding NIR measurements of the UFC being coupled thereto. It is for example possible to use CHN analysers which for example incinerate the UFC and by means thereof determine how much C and N is present. The dry matter content and/or the water content may also be determined by, for example, weighing the UFC, placing the UFC in an oven, and weighing the UFC again after it has spent, for example, 2 hours at 120°C in the oven. In this case, the calibration model is always well-adapted to the current circumstances, as a result of which the information on the F/U molar ratios of the UFC which is available is always accurate. Thus, it is for example possible for the NIR measurements to depend on the wear of the NIR measuring apparatus and/or the wear of materials/components which are used during UFC production and/or the contamination of the NIR measuring apparatus which occurs during use.

If both online NIR measurements and offline NIR measurements are performed at one said location, online NIR measurements and corresponding offline NIR measurements can be compared to each other. On the basis thereof, it is also possible to optimize the calibration models used, preferably to continuously optimize them, so that the calibration model is always well-adapted to the current circumstances. The calibration models used for the offline NIR measurements are preferably optimized by means of corresponding wet-chemical measurements of the UFC. The calibration models used for the online NIR measurements are preferably optimized by means of corresponding offline NIR measurements and/or wet-chemical measurements.

Preferably, at least one calibration model is used for every NIR measuring apparatus, preferably location-specific calibration models are also used for the locations. This ensures that an accurate calibration model is used for every NIR measurement. Thus, it is also possible to take the environmental conditions of every location into account.

Online NIR measurements at the first location may be performed by arranging a probe/sensor in the first line. This first line may consist of one or more lines and/or may comprise branches. A said line may consist of a main line and a bypass line to this main line, in which case the probe/sensor then preferably extends in this bypass line. With the aid of a bypass line, the circumstances of the UFC which comes into contact with the probe/sensor may be optimized, such as for example the temperature, the flow velocity, etc. In this way, the measurement conditions may be kept within desired ranges more efficiently in order thus to produce more accurate measurement results.

The F/U molar ratio of the resultant UFC is preferably between 4 and 6, more preferably between 4.5 and 5.5, still more preferably between 4.8 and 5.2 and most preferably between 4.9 and 5. The urea-formaldehyde concentrate is here regarded as a raw material for manufacturing urea-formaldehyde glue, as a result of which it is then also desirable for the resultant UFC to be as uniform as possible. When producing the desired urea-formaldehyde glue, it is then very readily possible to determine which quantities of each raw material are required.

In order to determine properties of the resultant formaldehyde concentrate, the molar ratio of F/U is determined here. Determining the F/U molar ratio may be carried out, for example, by directly converting the NIR measurements in F/U molar ratios using a calibration model. However, if the NIR measurements determine carbon (C) and nitrogen (N), it is also possible, instead of using calibration models which directly convert in F/U molar ratios, to use calibration models which determine the C content and the N content and then to determine the F/U molar ratio from these. The F/U molar ratio virtually corresponds to the formaldehyde/amines multiplied by two molar ratio. Instead of determining the F/U molar ratio with the aid of a calibration model, it is also possible to determine the formaldehyde/amines multiplied by two molar ratio . Here, the F/U molar ratio is then also considered to be a synonym for the formaldehyde/amines multiplied by two molar ratio. Obviously, it is then also possible to determine the formaldehyde/amines molar ratio.

Preferably, the UFC comprises between 18 and 22% by weight of water, more preferably between 19 and 21% by weight of water. If the NIR measurements determine the amount of C and the amount of N, it is also possible to determine the water content in addition to the F/U molar ratio, the formaldehyde content and the urea content. In this way, all information of the UFC, which is produced substantially and preferably only from formaldehyde, urea and water, is known. In this way, the correct dosing of the raw materials for producing the desired urea-formaldehyde glue is made simpler.

In a highly preferred embodiment, the UFC is produced in a continuous production process and this continuous production process is at least controlled on the basis of said NIR measurements of the UFC, preferably at least on the basis of said NIR measurements of UFC of the first location. Obviously, the production of UFC may also (additionally) be controlled on the basis of NIR measurements of UFC of the second location. These NIR measurements may be carried out online and/or offline. With the aid of NIR measurements, it is possible to verify if the F/U molar ratio of the resultant UFC deviates from the desired value and it is also possible to intervene quickly in the production process of UFC. Variations in the F/U molar ratio of the resultant UFC may consequently be reduced, resulting in a more constant quality and composition of the resultant UFC. Preferably, the NIR measurements of UFC of the first location are at least carried out online at the first location. Carrying out NIR measurements online makes it possible to know when there are variations in the F/U molar ratio more quickly. An additional advantage is that, since there are fewer variations in the F/U molar ratio, the determined F/U molar ratios are more accurate because a said calibration model is based on the desired F/U molar ratio. The greater the deviation, the greater the possible measurement error and the less suitable the calibration model is for determining the F/U molar ratio. Due to the fact that it is in this case possible to intervene in the production process to keep the composition of the resultant UFC as constant as possible, all NIR measurements can contribute to a more accurate determination of the F/U molar ratio, because the calibration models which are used are very suitable for determining the F/U molar ratio. If the production process of the UFC is modified at least on the basis of said NIR measurements of UFC of the first location, then preferably NIR measurements are carried out online as well as offline. In this case, the F/U molar ratio can be determined more accurately and, in addition, calibration models used at the first location can be optimized with the aid of online NIR measurements and/or offline NIR measurements. The determined F/U molar ratio may, for example, deviate by at most 0.02 from the actual F/U molar ratio, preferably by at most 0.01 and most preferably by at most 0.005. Problems with the UFC production, such as blockages of lines and/or other defects in supplying the components of UFC, can be detected quickly in this way and thus be solved.

Preferably, in order to form said urea-formaldehyde glue, the raw materials are dosed into the reactor on the basis of said F/U molar ratios of the UFC of the first location and/or the second location. The determined F/U molar ratios of the dosed UFC are very similar to the actual F/U molar ratios of the dosed UFC. Preferably, use is at least made of the determined F/U molar ratios of the UFC of the second location.

Preferably, NIR measurements are performed online on the UFC at least at the first location or the second location. In this way, the quality/properties of the UFC which is added to the reactor can be determined continuously and/or at desired points in time, as a result of which the dosing of the raw materials can take place in an optimum manner.

Furthermore preferably, NIR measurements of the formaldehyde concentrate of the first or the second location are also partly performed offline, with the calibration model for online NIR measurements being optimized by means of corresponding NIR measurements which were performed offline. This also makes it possible to optimize the calibration model during the production of the UFC. In this way, the calibration model can then, for example, take into account the wear which occurs during production and/or wear of the NIR measuring apparatuses and/or changes which occur as a result of maintenance and/or changes due to contamination, etc. The calibration model for online NIR measurements may additionally be optimized by means of corresponding wet-chemical measurements. The calibration model for online NIR measurements may also be optimized only on the basis of corresponding wet-chemical measurements.

Also preferably, NIR measurements of the UFC of the first location take place both online and offline, with the calibration model for online NIR measurements at the first location being optimized by means of said NIR measurements which were performed offline. Thus, the calibration model can then, for example, take into account the wear which occurs during production and/or the wear of the NIR measuring apparatuses and/or changes which occur as a result of maintenance and/or changes due to contamination, etc.

In a highly preferred embodiment, a said calibration model is at least formulated using NIR measurements of reference samples, wherein the calibration model is preferably optimized on the basis of NIR measurements performed during the manufacturing of UFC. With the aid of reference samples, it is possible to formulate a good calibration model for starting up NIR measurements during the manufacturing of UFC. This calibration model is formulated on the basis of NIR measurements and corresponding wet-chemical measurements. Wet chemistry, or general chemistry, describes a broad range of classical chemical analyses. With the aid of classical chemical analyses, the formaldehyde content, urea content, water content and F/U molar ratio of the reference samples are then also determined/confirmed, with the NIR measurements being coupled thereto. This calibration model can then be optimized continuously or at specific points in time so as to always have an up-to-date calibration model. The calibration model may be kept up to date with the aid of offline NIR measurements and/or online NIR measurements and wet-chemical measurements. It is also possible to replace some of said NIR measurements with other types of measurements or additional other types of measurements may be used in combination with said NIR measurements in order to increase the accuracy.

Preferably, at least two calibration models are used to determine the F/U molar ratio of the UFC, being at least one first calibration model which is used for NIR measurements on UFC of the first location and one second calibration model which is used for NIR measurements on UFC of the second location. Also in case offline and online NIR measurements for UFC of one said location are performed, a different calibration model is preferably used for the offline NIR measurements than for the online NIR measurements.

Preferably, the NIR measurements on UFC of the first location are carried out at a temperature of between 30°C and 50°C, for example between 35°C to 45°C and/or preferably the NIR measurements on UFC of the second location are carried out at a temperature of between 30°C and 40°C, for example 30°C to 35°C. The temperature at the first location usually differs from the temperature of the location. This is preferably taken into account with online NIR measurements and the calibration model is preferably adapted accordingly. For the offline NIR measurements, it is simpler to always measure at the same temperature, or in any case within a narrow temperature range, such as for example a temperature range of at most 2°C or at most 1°C.

If both online NIR measurements and offline NIR measurements are performed on UFC of a said location, it is also possible to take into account environmental parameters, such as for example the environmental temperature. Thus, depending on the season, or depending on the measured environmental temperature, it is possible to use the appropriate calibration model or to apply a suitable corrective factor when determining the F/U molar ratios of the UFC. Obviously, if the calibration model is constantly being optimized on the basis of performed NIR measurements, the abovementioned additional measures may be redundant.

The NIR measurements with the aid of an NIR measuring apparatus are preferably performed at wavelengths of between 1000 nm and 2200 nm, more preferably between 1100 nm and 1750 nm. At these wavelengths, very accurate calibration models may be drawn up.

In a preferred embodiment, the NIR measurements on UFC are performed with the aid of a probe through which the UFC flows, preferably through which the UFC flows at a flow velocity of between 0.05 m/s and 0.34 m/s, and that the NIR measurements with the aid of the probe are preferably NIR measurements via transmission. Instead of transmission, it is also possible to use transflection or reflection. In addition, the flow velocity is preferably between 0.20 m/s and 0.24 m/s. Instead of using a probe through which the UFC flows, use may also be made of a sensor past which the UFC flows. The viscosity of UFC allows the use of a probe for NIR measurements. If the UFC is being measured at the location of a bypass of a line or in a recirculation line of a storage tank, the flow velocity can be kept more constant, as a result of which the calibration model used can provide very accurate results with regard to the F/U molar ratio of the UFC.

In one specific embodiment, the second location is situated in the storage tank. The UFC produced is transferred to a storage tank. By performing NIR measurements in the storage tank, it is possible to optimize the UFC production on the basis thereof. In addition, the properties of the UFC which is dosed into the reactor are precisely known. The storage tank comprises at least one storage vessel, but may additionally also comprise recirculation lines and/or bypass lines and/or an agitator, etc. NIR measurements can then be performed on UFC of the storage vessel and/or of additional elements of the storage tank, online and/or offline. The more NIR measurements are performed, the more accurate the determined F/U molar ratios of the UFC can be and/or the more accurate the calibration models used can be.

In one specific embodiment, the amount of carbon and nitrogen is measured during the NIR measurements and the formaldehyde/urea molar ratio is determined directly or indirectly by means of the respective calibration model, and the content of formaldehyde, urea and water of the urea-formaldehyde concentrate is preferably additionally determined on the basis of the amount of carbon and nitrogen. By determining the carbon and nitrogen content, it is possible to determine the complete composition of the UFC. On the basis thereof, it is possible to add the other raw materials to the reactor in a very accurate way in order thus to obtain the desired urea-formaldehyde glue.

The raw materials may additionally comprise one or more raw materials from the list comprising: melamine and additives comprising for example acetic acid, sodium hydroxide and sodium acetate. Preferably, all raw materials are stored separately and are each dosed into the reactor separately by means of, for example, a separate dosing line. By using separate dosing lines, only minimal cleaning is required, for example only cleaning of the reactor and the lines which are situated downstream of the reactor if a different glue is to be produced. Melamine is preferably a white odourless powder which is usually produced from urea, and preferably has a water content of less than 0.1% by weight. Melamine (or 1,3,5-triazine-2,4,6-triamine) is a compound with C₃H₆N₆ as gross formula. Structurally, it consists of an aromatic ring of 3 carbon atoms and 3 nitrogen atoms.

In a preferred embodiment, the NIR measurements of the UFC of the second location are linked to the corresponding NIR measurements of the UFC of the first location, wherein use is made of at least one first calibration model for the NIR measurements of the urea-formaldehyde concentrate of the first location, and use is made of at least one second calibration model for the NIR measurements of the urea-formaldehyde concentrate of the second location, wherein at least one of the said calibration models is optimized on the basis of said linking of the NIR measurements.

If a deviation is found between a determined F/U molar ratio of the UFC of the second location and a said corresponding determined F/U molar ratio of the UFC of the first location which exceeds a determined threshold value, it may be suspected that one of the determined values is based on an incorrect measurement. It may then be decided not to use one of said determined F/U molar ratios or neither, and only to use values which have been determined earlier or later for dosing the raw materials into the reactor. All this may be performed automatically with the aid of, for example, a computer program. The deviation is preferably less than 0.2, more preferably less than 0.1, and most preferably less than 0.05. For a corresponding measurement, the time it takes to move the UFC from the first location to the second location is taken into account. When there are a sufficient number of NIR measurements, it is no problem if certain NIR measurements cannot be used.

Linking the NIR measurements can be done directly, but also indirectly by linking the F/U molar ratios.

In a highly preferred embodiment, the UFC is manufactured in a continuous production process which connects to the first line and in which use is made of at least two storage tanks, wherein the reactor is alternately filled from these at least two storage tanks, and wherein the first line alternately replenishes the two storage tanks, wherein preferably one of said storage tanks is always replenished, while the other storage tank is being emptied into the reactor. By using at least two storage tanks, the production in the reactor, which preferably takes place in batches, can proceed smoothly. Preferably, NIR measurements are then performed on UFC in all said storage tanks. These may be online and/or offline NIR measurements.

In a highly preferred embodiment, NIR measurements are carried out on the resultant urea-formaldehyde glue, wherein one or more characteristics of this urea-formaldehyde glue are determined on the basis of these NIR measurements and a respective calibration model. The quality of the resultant urea-formaldehyde glue can be ensured even more reliably. With the aid of these NIR measurements, it is possible to add one or more raw materials to the reactor if said characteristics deviate from a respective desired value, in order to obtain a urea-formaldehyde glue whose characteristics are closer to the desired characteristics. The urea-formaldehyde glue which then leaves the reactor is then also the desired urea-formaldehyde glue. Additionally, because the characteristics of the raw materials, and in particular the characteristics of the resultant UFC, are very well known, the risk of said characteristics, which were determined with the aid of said NIR measurements, deviating from the desired values is smaller. The latter makes it possible to produce more accurate calibration models for the urea-formaldehyde glue, which in turn results in a more accurate determination of the one or more characteristics. This also causes a significant reduction in the time required to manufacture the glues, as fewer corrections are required and/or virtually no additional dosing of raw materials is required. In addition, NIR measurements of the urea-formaldehyde glue may be coupled to the NIR measurements of the UFC, which in turn produces more accurate calibration models for NIR measurements of the UFC and for NIR measurements of the urea-formaldehyde glue.

If it is found that one or more of said characteristics of the urea-formaldehyde glue formed in the reactor deviate from the desired values, then it is also possible to opt to produce one or more other batches of urea-formaldehyde glue in the reactor on the basis of this first resultant urea-formaldehyde glue, and to mix these one or more other batches with the first resultant urea-formaldehyde glue in order thus to produce a glue having the desired characteristics.

In one specific embodiment, these one or more characteristics, for example the F/U molar ratio, of the urea-formaldehyde glue may be coupled to the F/U molar ratio of the corresponding UFC, so that the UFC production can be optimized on the basis of the NIR measurements of the urea-formaldehyde glue. In this way, the quality of the urea-formaldehyde glue can be linked to the quality of the UFC, production steps and the environmental parameters. The optimisation of the production steps and/or the environmental parameters may be effected with the aid of a computer, for example with the aid of an algorithm and/or artificial intelligence. In this case, it is possible to use a self-learning model and parameters may be shown/communicated to an operator, so that any intervention on the basis of NIR measurements is possible.

These NIR measurements on the urea-formaldehyde glue may be carried out on urea-formaldehyde glue of different locations and the NIR measurements may be performed online and/or offline. Locations may, inter alia, comprise the reactor, one or more lines which are situated downstream from the reactor and, for example, lead to a storage tank of the resultant urea-formaldehyde glue, one or more storage tanks of the resultant urea-formaldehyde glue, one or more lines which extend downstream of a said storage tank. Preferably, when choosing the calibration model, one or more of the following factors are taken into account:
- the various locations of the urea-formaldehyde glue because, once produced, the urea-formaldehyde glue may continue to react and may thus, for example, become more viscous;
- online or offline measurement and also the possible measurement conditions;
- the type of urea-formaldehyde glue, for example a UF glue or an MUF glue and the desired amounts of raw materials for the type of urea-formaldehyde glue.

Furthermore preferably, one or more characteristics of the glue are selected from the group comprising: F/U molar ratio, viscosity, dry matter content, pH, gelling time, melamine content, and dynamic differential calorimetry data. If the F/U molar ratio is determined, and preferably also other said characteristics are determined, and more preferably all said characteristics are determined, the production process of the substrate, for example the dosing of glue, the pressing speed, pressing temperature, the addition of additives, etc. can be regulated very accurately, as a result of which the quality and the uniformity of the resultant substrates is very good. For boards which comprise additional layers apart from the substrate, for example boards comprising substrate and one or more layers laminated onto the substrate, high-quality and uniform substrates ensure that laminating can take place under optimum circumstances. By means of this method, high-quality and uniform boards are then formed. The calibration models for these one or more characteristics are preferably compiled with the aid of wet-chemical measurements and NIR measurements of reference samples and are furthermore preferably optimized with the aid of NIR measurements and/or wet-chemical measurements of urea-formaldehyde glue which are performed during production. Below, there follows a non-exhaustive list of useful wet-chemical measurements: viscosity may be measured with the aid of a viscosity meter, dry matter content with the aid of an oven, pH with a pH meter, dynamic differential calorimetry data with a DSC oven, gelling time may be determined by placing urea-formaldehyde glue together with a hardener - for example ammonium nitrate - into a test tube and to put this combination in boiling water and to determine when gelling takes place - for example by using of a pendulum that can move up and down.

Furthermore preferably, the produced urea-formaldehyde glue is transferred via at least one line from the reactor to a glue storage tank, wherein NIR measurements of the urea-formaldehyde glue are performed on urea-formaldehyde glue of at least one of the following locations: the reactor, the last-mentioned line or the glue storage tank. A said glue storage tank may be the tank which is used to unload the urea-formaldehyde glue in, for example, a vehicle, such as a lorry or a ship, or for unloading in the production environment of said substrates. When a said cargo tank is present, it is also possible to measure the urea-formaldehyde glue of the cargo tank. In this way, it is possible to know the characteristics of the actual urea-formaldehyde glue which is used for producing the substrate very well, which then obviously contributes to the manufacture of high-quality and uniform boards. Also, use may be made of a cooling tank which extends, for example, between the reactor and the storage tank/cargo tank. This cooling tank may also be regarded as a storage tank. Obviously, NIR measurements may be performed on the urea-formaldehyde glue in this cooling tank. There may be several storage tanks and/or cargo tanks and/or cooling tanks.

In a highly preferred embodiment, NIR measurements are carried out on the urea-formaldehyde glue in the production environment of the substrate, so that the properties of the used formaldehyde glue are very well known during the course of producing the substrate. Changes due to transportation, age of the urea-formaldehyde glue, etc. have then been taken into account here.

Furthermore preferably, NIR measurements of the urea-formaldehyde glue are carried out on urea-formaldehyde glue in the reactor, and that for said one or more characteristics reference ranges are defined within which these characteristics have to be, and wherein, if at least one of said characteristics is outside its reference range, one or more raw materials are added to the reactor before the urea-formaldehyde glue leaves the reactor, in order thus to optimize the urea-formaldehyde glue. It is thus possible to intervene in a very suitable way during the production of the urea-formaldehyde glue if it is found that the urea-formaldehyde glue will not reach the desired values without additional quantities of certain raw materials.

Various types of urea-formaldehyde glues may be produced. This not only refers to the division into, for example, UF glue and MUF glue, but also denotes the fact that different types of UF glue and different types of MUF glue may be produced. Which type of urea-formaldehyde glue is produced depends on the desired type of end product. Thus, the end product may be a particleboard having a certain thickness and/or density and/or water resistance and/or fire resistance, a certain number of layers, etc. or an MDF/HDF having a certain thickness and/or density and/or water resistance and/or fire resistance, etc. or the end product may be a melaminated substrate - for example a particleboard or MDF/HDF - in which one or more impregnated layers, such as paper layers, are laminated onto one or both sides of the substrate. In this case, it is possible to produce the most suitable urea-formaldehyde glue for every type of end product in order thus to achieve high-quality and uniform end products.

The NIR measurements on the glue may in this case be carried out at wavelengths of between 900 nm and 1900 nm.

Manufacturing of the substrate takes place on the basis of said one or more characteristics of the glue, wherein for example production parameters, such as pressing time, pressing temperature, amount of applied glue, are adjusted and/or additional raw materials, such as formaldehyde catchers and/or additional urea and/or raw material comprising an ammonium, such as ammonium nitrate or ammonium phosphate, are added when coating the wood particles with glue. During the production of the substrate, formaldehyde may be extracted. The degree of extraction may be adjusted on the basis of the said one or more characteristics of the glue.

Furthermore, regarding the manufacturing of the substrate, there is a reference range for the F/U molar ratio of the urea-formaldehyde glue, wherein, if the F/U molar ratio of the urea-formaldehyde glue is outside this range, the production parameters are adjusted and/or additional raw materials are added. For urea-formaldehyde glues, the F/U molar ratio is usually between 0.8 and 1.4. Each type of urea-formaldehyde glue preferably has a reference range in which the difference between the highest and the lowest value of this reference range is at most 0.1, more preferably is at most 0.04, and most preferably is at most 0.02. In this way, a stable and uniform production is possible.

Preferably, NIR measurements are carried out on the urea-formaldehyde glue at a location upstream of where the wood particles are coated with glue and downstream of the point where it leaves the storage tank, in order to determine one or more characteristics of the urea-formaldehyde glue which is used in the manufacture of the substrate. In this way, said one or more characteristics of the urea-formaldehyde glue may be very well known at the start of the production, which contributes to the uniformity and the quality of the resultant substrate. Furthermore preferably, these characteristics of the urea-formaldehyde glue at this said location are coupled to the characteristics of the urea-formaldehyde glue at other locations, for example locations in the glue production, in order thus to optimize the quality of the urea-formaldehyde glue and/or to optimize the calibration models. On the basis of these NIR measurements, it is also possible to opt for the addition of additional raw materials, such as additives - formaldehyde catchers -, hardeners - ammonium nitrate or ammonium phosphate -, urea, etc. to the wood particles when coating the wood particles with glue.

The type of glue used to produce a substrate preferably depends on the board to be produced, wherein the calibration model used for the NIR measurements of the urea-formaldehyde glue then at least depends on the type of glue. In order to determine the F/U molar ratio (or formaldehyde/amines multiplied by two) by NIR measurements and/or other said characteristics, good calibration models are required. Since infrared absorption/reflection/... etc. depend greatly on the type of glue, it is then also desirable to have a calibration model for each type of glue and/or characteristic. Better yet, even for the same type of glue, it may be useful to have several calibration models, since the measurement conditions at different locations may be different and/or the urea-formaldehyde glue also continues to react after production. The more accurate the calibration model, the more accurate the specific characteristic.

Preferably, the type of glue used to produce a substrate depends on the board to be produced, in which case the calibration model used for the NIR measurements of the urea-formaldehyde glue depends at least on the type of glue. Thus, there may be one or more differences for particleboard, MDF and HDF, for the substrate from the list: density, water resistance, fire resistance, strength, composition of the wood particles, etc.

In a highly preferred embodiment, the wood particles are coated with glue during the manufacturing of the substrate by means of at least said urea-formaldehyde glue, wherein NIR measurements are carried out on the glue-coated wood particles, preferably online NIR measurements, to determine the reactivity of the glue-coated wood particles. By determining the reactivity, the pressing parameters can be accurately adapted to the properties of the glue-coated wood particles and/or additives may also be added when coating the wood particles with glue in order, for example, to increase or decrease the reactivity. The reactivity is determined to a very great degree by the amount of formaldehyde. Thus, wood particles originating from the recycling of wood waste may already have a high formaldehyde content. The reason for this is that wood waste may comprise, for example, particleboard and/or wood fibreboard which were produced using urea-formaldehyde glues. At relatively high amounts of formaldehyde, i.e. at relatively high molar ratios of formaldehyde to urea, the glue-coated wood particles are more reactive and it will be possible to press more quickly. However, this may also result in an excess of formaldehyde emission not only during production, but also during use of these boards. This may be solved, for example, by adding formaldehyde catchers when coating the wood particles with glue and/or before the pressing operation, and/or by using a urea-formaldehyde glue with a lower formaldehyde content. For example, if substrates are manufactured with a certain amount of recycled wood particles, it is possible to use a urea-formaldehyde glue which is modified for this purpose. If the reactivity is too low, it is possible to slow down the pressing operation, but it is also possible to add hardeners, such as ammonium nitrate or ammonium sulphate, when coating of the wood particles takes place in order thus to increase the reactivity. On the basis of these NIR measurements, it is also possible to determine whether the formaldehyde emissions of these substrates do not exceed any threshold values. On the basis of these NIR measurements, it is preferred if one or more of the below adjustments are made in the production process. If the reactivity is too high, i.e. when the formaldehyde content is excessively high, for example a molar ratio of formaldehyde to urea is too high: add further formaldehyde catchers (additives), use another glue, use fewer wood particles from a certain source or do not use them at all. If it is found that the reactivity is too low, i.e. when the formaldehyde content is excessively low, for example a molar ratio of formaldehyde to urea is too low, it is for example possible: to slow down the production process, to add less urea, to use a different glue, or to use wood particles which already comprise a certain amount of formaldehyde. When reactivity is low, but not to such a degree that it is too low for a good production, it is for example also possible to opt for using (more) formaldehyde-contaminated wood particles from recycling.

In a highly preferred embodiment, the wood particles at least partly originate from a waste product comprising wood components, preferably originate to at least 60% by weight from this waste product, more preferably at least 70% by weight, most preferably at least 90% by weight. Preferably, online NIR measurements and/or offline NIR measurements of this waste product are carried out for separating suitable wood particles from non-wood particles and/or contaminated particles - for example wood particles with a high degree of chemical pollution -, wherein this waste product is preferably comminuted and said online NIR measurements are at least carried out on this comminuted waste product.

This waste product is, for example, designated as wood waste and may, for example, be end-of-life particleboard, such as furniture panels and the like, or end-of-life wooden products, such as wooden windows and the like, or end-of-life HDF and MDF, end-of-life laminate, or production waste. This wood waste is then for example comminuted/fragmented/shredded in order to form, for example, chips which may be used in the production of (new) particleboard. HDF and MDF may for example be converted to recycled fibres which are useful in the production of new MDF/HDF substrates. Obviously, this wood waste comprises many contaminants, such as plastic, metal, harmful paints/lacquers/etc. Also, recycled wood particles may emit more formaldehyde than is permitted and this in particular because the standards for formaldehyde have been tightened over the past few years and this wood waste may originate from a period in which a much higher formaldehyde emission was permitted. Ways of removing certain contaminants already exist, but most of these require large amounts of energy and/or allow for too many contaminants (or conversely remove too many useful particles). Surprisingly, it has been found that contaminants and/or highly contaminated particles can readily be separated with the aid of online NIR measurements, as a result of which the particles which remain are substantially usable wood particles. With the aid of NIR measurements, it is possible to obtain wood particles of a better quality and also more uniform wood particles which can then, together with a better-quality glue, provide a better-quality and more uniform substrate.

Furthermore preferably, the comminuted waste product is transferred to a cleaning installation with the aid of a conveyor belt, wherein the comminuted waste product is subjected to said NIR measurements in this cleaning installation, wherein said contaminants and/or highly contaminated particles are detected by means of said NIR measurements and are separated off. The quality of the resultant particles, i.e. the resultant wood particles, is markedly better in this case than when this production step is omitted. Preferably, the comminuted waste product has a moisture content of between 8% wt and 30% wt, for example between 10% wt and 15% wt, when it is subjected to said NIR measurements.

In one specific embodiment, the wood particles at least partly originate from a waste product comprising formaldehyde, wherein these wood particles are subjected to NIR measurements in order to determine the formaldehyde content in these wood particles, and that both the F/U molar ratio of the used urea-formaldehyde glue and the formaldehyde content of said wood particles are taken into account when manufacturing the substrate. The wood particles originate, for example, from recycled boards comprising/consisting of particleboard or recycled boards comprising/consisting of MDF/HDF. To determine the formaldehyde content, use is then made of one or more calibration models. Thus, there may be a calibration model to be used for each recycling source. In addition, it is possible to take into account other characteristics of the glue which have been mentioned above. Preferably, the formaldehyde content is determined when the wood particles have a moisture content of between 8% wt and 15% wt, for example between 10% wt and 12% wt.

In a highly preferred embodiment, non-destructive online measurements are carried out after the substrate has been pressed in order to determine whether the resultant boards satisfy desired quality standards. These measurements may be coupled to other measurements in order thus to optimize the production process. These non-destructive online measurements may comprise one or more of the following measurements: NIR measurements, measurements using radio waves, visual measurements. These measurements may determine one or more of the following: density, formaldehyde emission and thickness.

In one specific embodiment, the substrate is a particleboard, wherein, optionally, a decorative layer is applied to the substrate, wherein furthermore preferably the application of the decorative layer on the substrate takes place on the basis of the said one or more characteristics of the glue which was used to manufacture the substrate.

The substrate may be a single-layer substrate, but may, for example, also have multiple layers. Often, particleboard has three layers, being two outer cover layers and a core layer, wherein coarser wood chips are used for the core layer and finer wood chips are used for the cover layers. When producing multi-layer substrates, the same urea-formaldehyde glue may be used for every layer, but the urea-formaldehyde glue used may also depend on the layer to be produced. Preferably, alle used urea-formaldehyde glues are produced as indicated above. Obviously, only one layer may or not all layers may comprise a urea-formaldehyde glue produced as described above. It is even possible for one or more layers to not comprise any urea-formaldehyde glue and, for example, to comprise a different type of glue, such as an isocyanate glue or a bio-based glue.

The substrate may be provided with a fire-retardant layer and/or an electricity-conducting layer and/or water-repellent layer in order to form the board. Also, when mixing the wood particles and the glue, fire-retardant components and/or electrically conductive elements and/or water-repellent elements may be added in order thus to obtain a substrate comprising these components.

In one specific embodiment, the substrate is an MDF/HDF, and wherein, optionally, a decorative layer is applied to a substrate, wherein furthermore preferably the decorative layer is applied to the substrate on the basis of said one or more characteristics of the glue which was used to manufacture the substrate.

The present invention relates to a method for producing urea-formaldehyde glue and is defined in claim 1. Preferred embodiments are described in the dependent claims. All features and embodiments with regard to the production of the urea-formaldehyde glue described in the above method for producing boards are applicable to this method for producing urea-formaldehyde glue.

The present disclosure also relates to a method for producing other glues suitable for producing the abovementioned substrates, wherein these other glues are not based on urea and formaldehyde. The principle in which use is made of NIR measurements for optimization of the glue, as described above, is also useful for these other glues. With the aid of the NIR measurements, other/additional important properties of this glue may then be measured.

In order to show the features of the invention in more detail, some preferred embodiments are described below by way of example and without being limited thereto, with reference to the accompanying drawings, in which:
- ***Fig. 1*** is a diagrammatic representation of the production of a urea-formaldehyde glue;
- ***Fig. 2*** is a diagrammatic representation of the production of a urea-formaldehyde concentrate;
- ***Fig. 3*** is a diagrammatic representation of the production of wood-based substrates.

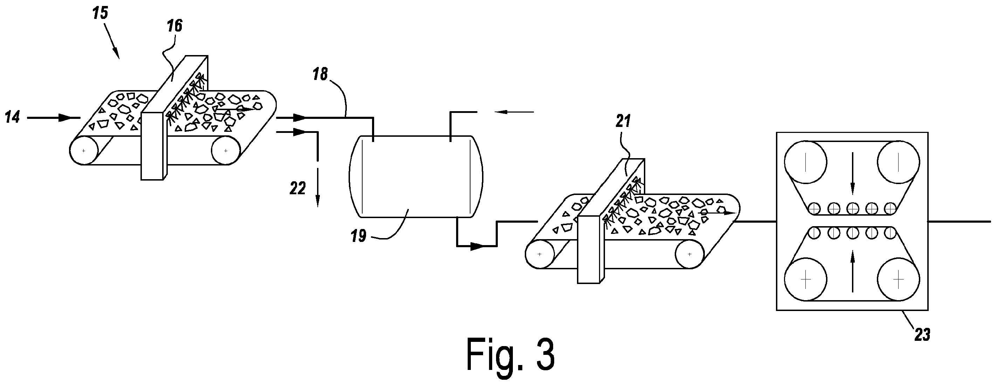

The figures diagrammatically show production steps of a method for producing boards. The boards which may be produced in this case comprise at least a wood-based substrate, such as particleboard or MDF/HDF. Optionally, the boards may comprise one or more layers which are applied to one side or to both sides of the substrate. These layers may, for example, be resin-impregnated paper layers, comprise a veneer, but may, for example, also be a coating, a lacquer, a film - such as a thermoplastic film -, a varnish, etc. The figures do not show the application of such one or more layers. Only the production steps for the production of the substrate are shown. If the boards only comprise a substrate, there may still be additional production steps, such as, for example, the sanding of the substrate, the production of coupling parts, etc. All this is also not shown in Fig. 3.

The most important raw materials for producing the substrate are the wood particles and the urea-formaldehyde glue. Therefore, particular emphasis is placed on these in the figures.

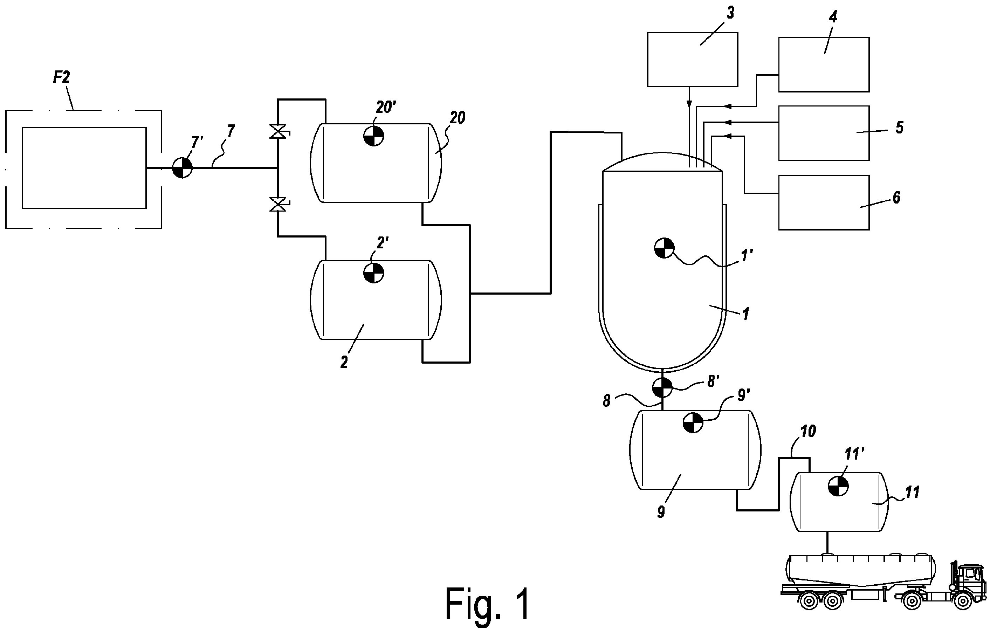

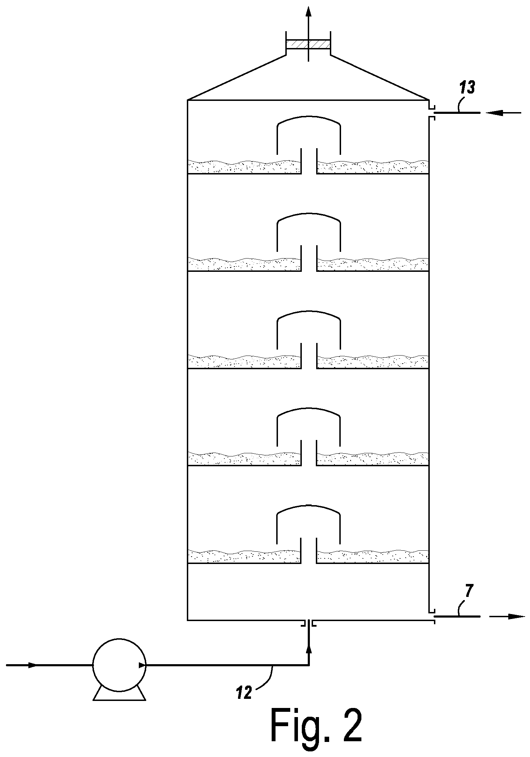

Fig. 1 diagrammatically shows a possible embodiment for producing the urea-formaldehyde glue. Fig. 2 shows a possible embodiment for producing UFC. UFC is a raw material for producing the urea-formaldehyde glue. The production of UFC is diagrammatically illustrated in Fig. 1 by the rectangle surrounded by F2, and in this way refers to Fig. 2 as a possible embodiment. Fig. 3 diagrammatically shows an embodiment of the production of the substrate, wherein the production of the urea-formaldehyde glue is not illustrated, since this is already shown in Figs. 1 and 2.

As can be seen in Fig. 1, the urea-formaldehyde glue is formed in batches in a reactor 1. The raw materials of the urea-formaldehyde glue are dosed into this reactor 1 and mixed in order to produce the urea-formaldehyde glue. To this end, the reactor 1 is, by means of dosing devices, connected to storage units 2, 20, 3, 4, 5, 6 which respectively comprise UFC, UFC, urea, melamine, water and additives. Here, the additives are represented by one frame 6, but in reality each additive has a separate storage unit, such as a storage tank or the like. The UFC is prepared in a continuous production process and is stored in at least two storage tanks 2, 20. By using at least two storage tanks 2, 20, it is readily possible to switch from a continuous production process to a batch production process, in which dosing takes place from the one storage tank 2, 20 while the other storage tank 20, 2 can be replenished. If MUF glues are to be produced, dosing also takes place from the storage unit 4 comprising melamine. When producing any type of urea-formaldehyde glue, UFC is always dosed from a UFC storage tank 2, 20, urea from a urea storage tank 3, and water from a water storage tank 5 or a water pipe connected to the mains system.

Fig. 2 shows the continuous production process of UFC. The UFC is produced by bringing formaldehyde gas into contact with urea water, being water comprising urea, for example water comprising between 56 and 58% by weight of urea. This is carried out, for example, in a vertical upright absorber with an inlet 12 for formaldehyde gas at the bottom and an inlet 13 for urea water at the top. At the bottom, the resultant UFC is then tapped off via a first line 7 and transferred to a respective UFC storage tank 2, 20. This illustrated absorber comprises 'bubble caps'. However, it is also possible to use systems which use valves and the like. In order to be able to produce high-quality urea-formaldehyde glues, it is important that the composition of the UFC is as identical as possible, i.e. is as uniform as possible. This is achieved, inter alia, by performing NIR measurements on UFC of at least three locations. A first location 7' is the first line 7. The first line 7 may comprise one main line and additional branches or bypass lines. A second location 2' is a said UFC storage tank 2 and a third location 20' is the other said UFC storage tank 20. These UFC storage tanks 2, 20 may comprise recirculation lines and/or bypass lines and/or an agitator. Preferably, both online and offline NIR measurements are performed at all locations. For the online measurement, a probe is placed in a spot at the location where the UFC then flows through the probe. For the offline measurement, a sample of tapped UFC is introduced into an offline measuring apparatus. The measurement conditions offline are easier to keep constant. For the offline measurements, the UFC can be tapped off at virtually the same spot where the online measurement is taking place, preferably both spots are as close to each other as possible, so that the offline and the online measurements can be correlated with each other. The online measurement for the first line 7 is carried out, for example, in a bypass of the main line in order to keep the measurement conditions as constant as possible. For the sake of simplicity (and because they are close to each other), Fig. 1 shows the spots for the online and offline measurements as being the same location 7', 2', 20'. With the aid of the NIR measurements, the carbon and nitrogen content are determined and by means of a suitable calibration model and optionally a calculation by means of the calibration model, the F/U molar ratio is then determined, as is the actual amount of urea, formaldehyde and water. Separate calibration models are used for the online measurements and the offline measurements. The calibration models are also location-dependent. All calibration models are produced with the aid of reference samples, and NIR measurements and wet-chemical measurements on these reference samples. In this case, it is important that the calibration models are adjusted continuously or at desired points in time on the basis of performed NIR measurements and corresponding wet-chemical measurements. Thus, the calibration model for the online measurements is adjusted, for example, on the basis of the corresponding offline NIR measurements and/or wet-chemical measurements, and the offline NIR measurements may be adjusted on the basis of wet-chemical measurements. Also, the NIR measurements of various locations may be used to adjust the calibration models. The F/U molar ratio and the water content, formaldehyde content and urea content are then also always highly accurate. The measurement error is preferably lower than 0.005. The production of the UFC is also adjusted on the basis of the NIR measurements. If it is found that there is too little formaldehyde, it is possible, for example, to dose more formaldehyde gas and/or to dose less urea. Also, problems in the production, for example blockages of supply lines, may be detected. If it is found that there is too much formaldehyde, it is possible, for example, to dose less formaldehyde gas and/or to dose more urea. As the measurements are online in this case, it is possible to respond very quickly with regard to the production of the UFC, as a result of which the UFC produced will also be much more uniform, which in turn leads to improved calibration models, and this in turn leads to more correct adjustments in the UFC production. The result is then also that a very uniform UFC raw material is produced for the urea-formaldehyde glue production.

In the reactor 1, various urea-formaldehyde glues may be produced. Thus, it is for example possible to produce UF glues or MUF glues. The desired board determines the urea-formaldehyde glue to be produced. If it is desired to produce a certain type of urea-formaldehyde glue with a certain composition, the desired raw materials are appropriately dosed into the reactor 1 and mixed. Thereafter, the resultant formaldehyde glue leaves the reactor 1 via at least one line and is transferred to a glue storage tank/glue cooling tank 9. From this glue storage tank/glue cooling tank 9, the glue is transferred to a glue storage tank/cargo tank 11 via a line 10. From this glue storage tank/cargo tank 11, the urea-formaldehyde glue can be pumped into a vehicle, as is illustrated here, a lorry, or it may be taken directly to the production environment of the substrate.

NIR measurements are also performed during the production of the urea-formaldehyde glue and on the produced urea-formaldehyde glue. With the aid of these NIR measurements, various characteristics are recorded. At least the following characteristics are recorded: formaldehyde/urea molar ratio, viscosity, dry matter content, pH, gelling time, melamine content, and dynamic differential calorimetry data. With the aid of these characteristics, it is possible to reliably predict how the urea-formaldehyde glue will behave in the production of a substrate. During the production of the substrate, wood particles are coated with, inter alia, the urea-formaldehyde glue and the glue-coated wood particles are compressed. Important pressing parameters are inter alia the temperature, pressure and pressing time. With the aid of the abovementioned characteristics, it is possible to predict how the glue-coated wood particles will behave during compression.

With regard to the urea-formaldehyde glue, NIR measurements are performed before it is used in the production of the substrate, in at least 3 locations 1', 9', 11'. These locations 1', 9', 11' are situated in the reactor 1, the glue storage tank/glue cooling tank 9 and the cargo tank/glue storage tank 11. A measurement may also be performed at a location 8' in the line 8. Preferably, at least offline measurements are carried out for all locations.

Online measurements may also be performed. For the online measurements, a probe may, for example, be placed in a spot at the location with the urea-formaldehyde glue then flowing through the probe. For the offline measurements, a sample of urea-formaldehyde glue is tapped and transferred to an offline measuring apparatus. The offline measurement conditions are easier to keep constant. For the offline measurements, the urea-formaldehyde glue may be tapped at virtually the same spot where the online measurement was performed, preferably both spots are as close as possible to each other, so that the offline and the online measurements can be correlated to each other. For the sake of simplicity (and because they are close to each other), the spots for the online and offline measurements are shown as being the same location 1', 8' and 10' in the figure. Separate calibration models are used for the online measurements and the offline measurements. The calibration models are also location-dependent. All calibration models are produced with the aid of reference samples. If it is found that the resultant urea-formaldehyde glue in the reactor 1 deviates from desired parameters, one or more of said raw materials are dosed into the reactor 1 again and the urea-formaldehyde glue only leaves the reactor 1 when the desired parameters have been met. This may take place quickly, as online NIR measurements are also performed in the reactor 1. Another possibility is to manufacture a new batch of urea-formaldehyde glue in the reactor 1 and to then mix this with the urea-formaldehyde glue which has one or more deviating parameters, in order thus to obtain a urea-formaldehyde glue which does have the desired parameters.

Fig. 3 diagrammatically shows the production of the substrate. The wood particles may be recycled wood particles which originate entirely from the recycling of wood waste, but they may also comprise fresh wood particles originating from fresh wood. Wood waste which is suitable for producing new particleboard is, in particular, used particleboard, waste originating from particleboard production, products comprising particleboard, such as wall panels or furniture panels, solid wood waste. Wood waste which is suitable for producing new MDF/HDF is, in particular, used MDF/HDF, waste from the MDF/HDF production, products comprising MDF/HDF, such as floor panels, wall panels and furniture panels, or solid wood waste. This wood waste may already comprise wood particles which have been coated with urea-formaldehyde glue, as a result of which this wood waste already has a degree of formaldehyde content.

In order to produce the substrate, the wood particles are coated with at least the urea-formaldehyde glue and the glue-coated wood particles are compressed to form a board shape.

From an ecological point of view, it is very interesting to use wood waste for producing new boards. However, the formaldehyde content of this wood waste may cause problems. In addition, contamination of wood waste, such as textiles, metal, plastic, other foreign materials, but also chemical contamination and other contaminants, may be problematic with regard to the quality of the boards produced using wood waste. It is therefore also important to use urea-formaldehyde glue which is suited to the properties of the wood particles used and it is also important to remove contaminants as much as possible.

By means of the disclosure, a large part of the contaminants may be removed, so that the recycled wood particles are of as high a quality as possible. The properties of the wood particles may also be taken into account. To this end, use is made, inter alia, of NIR measurements and any other measurements. If the wood particles comprise recycled wood particles, then NIR measurements on the wood waste are performed during the production of the recycled wood particles. This may be done directly on the wood waste, with suitable wood waste being separated from highly contaminated wood waste and non-wood waste, such as metal waste, plastic waste, by means of NIR measurements. Preferably, the wood waste is comminuted and this comminuted wood waste is transferred to a cleaning installation 15 in order to perform a cleaning step. This cleaning installation 15 comprises a conveyor belt 14 on which the comminuted wood waste is spread out across the width, with NIR measurements being performed with the aid of NIR spectrophotometer 16 which covers the entire width of the belt. Contaminations, such as plastic, textile etc., are detected with the aid of the NIR measurements and then removed from the conveyor belt with the aid of compressed air by means of a separate flow 22.

Preferably, there are still some additional steps for producing the wood particles which are performed before or after this cleaning step, depending on the substrate which is being produced. Thus, there may still be a drying step, a mixing with wood particles originating from fresh wood, separating according to size, a step for producing fibres, etc. The suitable recycled wood particles are then transferred to the glue coater 19 via a flow 18, optionally directly from the cleaning installation 15.

In order to produce the substrate, the wood particles and the urea-formaldehyde glue are brought together in a glue coater 19. Depending on the characteristics of the urea-formaldehyde glue and the wood particles and depending on the desired substrate, other additives may still be introduced into the glue coater 19, such as additional urea, formaldehyde catchers, hardeners, such as ammonium nitrate or ammonium sulphate, but also components to increase the fire resistance and/or electrical conductivity and/or water repellency.

Preferably, NIR measurements are performed on the urea-formaldehyde glue which is present in the production environment of the substrate, so that the parameters of the used urea-formaldehyde glue are known well. Here, it is important that NIR measurements are also carried out on the glue-coated wood particles, for example in the glue coater 19 or preferably on the glue-coated wood particles which exit the glue coater 19 and are headed for the spreaders of the glue-coated wood particles, and from there to the press 23 in order to be compressed to form a board shape. Thus, the glue-coated wood particles may be conveyed to the spreader with the aid of a conveyor belt, this spreader then being configured, for example, to scatter three layers of glue-coated wood particles on top of each other, after which the three layers of glue-coated wood particles are conveyed to the press 23. Preferably, NIR measurements are carried out on the glue-coated wood particles with the aid of an NIR spectrophotometer 21 at the location of the conveyor belt. With the aid of the NIR measurements, the reactivity of the glue-coated wood particles is determined. The reactivity is in particular determined by the formaldehyde content, therefore the amount of (free) formaldehyde or the F/U molar ratio can be determined with the aid of the NIR measurements and a calibration model. The reactivity is also determined by the amount of (free) formaldehyde in the wood particles. Modifications may be carried out on the basis of the NIR measurements of the glue-coated wood particles at the location of the glue coater 19. Thus, further additives may be added and/or a certain urea-formaldehyde glue may be opted for and/or other wood particles may be used, if it is found that the measured values deviate from the desired values. Another possibility or an additional possibility is to adjust the pressing parameters, such as pressing time, pressing temperature and pressing pressure. The amount of extraction may also be adjusted.

Preferably, an additional inspection step is carried out after the pressing operation. Thus, the density or other characteristics may for example be determined via non-destructive measurements and the pressing parameters may be adjusted on the basis thereof.

## Claims

1. Method for producing a urea-formaldehyde glue, wherein raw materials of this glue are combined in a reactor (1), wherein these raw materials at least comprise urea, water and a urea-formaldehyde concentrate, wherein the urea-formaldehyde concentrate is produced by combining urea, formaldehyde and water, and the resultant urea-formaldehyde concentrate is introduced into a storage tank (2, 20) with the aid of a first line (7), wherein the urea-formaldehyde concentrate is introduced into the reactor (1) from the storage tank (2, 20) with the aid of a second line, **characterized in that** formaldehyde/urea molar ratios of the urea-formaldehyde concentrate are determined on the basis of NIR measurements and at least one calibration model, and **in that** these NIR measurements are performed on urea-formaldehyde concentrate in at least two locations, being a first location which is situated in the first line (7) and a second location which is situated in the storage tank (2, 20) or the second line, and wherein the raw materials are dosed into the reactor (1) on the basis of said formaldehyde/urea molar ratios of the urea-formaldehyde concentrate.

2. Method in accordance with Claim 1, wherein the urea-formaldehyde concentrate is produced in a continuous production process and this continuous production process is at least controlled on the basis of said NIR measurements of the urea-formaldehyde concentrate, preferably at least on the basis of said NIR measurements of the urea-formaldehyde concentrate of the first location.

3. Method in accordance with Claim 1 or 2, wherein the raw materials are dosed into the reactor (1) on the basis of said formaldehyde/urea molar ratios of the urea-formaldehyde concentrate of the first location and/or the second location.

4. Method in accordance with one of the preceding claims, **characterized in that**, at least at the first and/or the second location, NIR measurements on the urea-formaldehyde concentrate are performed online.

5. Method in accordance with Claim 4, **characterized in that** NIR measurements of the urea-formaldehyde concentrate of the first and/or the second location are also partly performed offline, wherein the calibration model for online NIR measurements is optimized by means of corresponding NIR measurements which were performed offline.

6. Method in accordance with one of the preceding claims, **characterized in that** a said calibration model is at least formulated using NIR measurements of reference samples, and wherein the calibration model is preferably optimized on the basis of NIR measurements performed during the manufacturing of the urea-formaldehyde concentrate.

7. Method in accordance with one of the preceding claims, **characterized in that** at least two calibration models are used to determine the formaldehyde/urea molar ratios of the urea-formaldehyde concentrate, being at least one first calibration model which is used for NIR measurements on urea-formaldehyde concentrate of the first location and one second calibration model which is used for NIR measurements on urea-formaldehyde concentrate of the second location.

8. Method in accordance with one of the preceding claims, wherein the NIR measurements on urea-formaldehyde concentrate are performed with the aid of a probe through which the urea-formaldehyde concentrate flows, preferably through which the urea-formaldehyde concentrate flows at a flow velocity of between 0.05 m/s and 0.34 m/s, and that the NIR measurements with the aid of the probe are preferably NIR measurements via transmission.

9. Method in accordance with one of the preceding claims, wherein the raw materials additionally comprise one or more raw materials from the list comprising: melamine and additives such as acetic acid, sodium hydroxide and sodium acetate.

10. Method in accordance with one of the preceding claims, wherein the NIR measurements of the urea-formaldehyde concentrate of the second location are linked to the corresponding NIR measurements of the urea-formaldehyde concentrate of the first location, wherein use is made of at least one first calibration model for the NIR measurements of the urea-formaldehyde concentrate of the first location, and use is made of at least one second calibration model for the NIR measurements of the urea-formaldehyde concentrate of the second location, wherein at least one of the said calibration models is optimized on the basis of the said linking of the NIR measurements.

11. Method in accordance with one of the preceding claims, wherein the urea-formaldehyde concentrate is manufactured in a continuous production process which connects to the first line (7), and that use is made of at least two storage tanks (2, 20), wherein the reactor (1) is alternately filled from these at least two storage tanks (2, 20), and that the first line (7) alternately replenishes the two storage tanks (2, 20), wherein preferably one of the said storage tanks (2, 20) is always replenished, while the other storage tank (20, 2) is being emptied into the reactor (1).

12. Method in accordance with one of the preceding claims, wherein NIR measurements are carried out on the resultant urea-formaldehyde glue, wherein one or more characteristics of this glue are determined on the basis of these NIR measurements and a respective calibration model.

13. Method in accordance with Claim 12, wherein producing the substrate takes place on the basis of the said one or more characteristics of the urea-formaldehyde glue, wherein for example production parameters, such as pressing time, pressing temperature, amount of applied glue, are adjusted and/or additional raw materials, such as formaldehyde catchers and/or additional urea and/or hardeners, such as ammonium nitrate, are added when coating the wood particles with glue.

## Patentansprüche

1. Verfahren zur Herstellung eines Leims auf Harnstoff-Formaldehyd-Basis; wobei die Rohstoffe für diesen Leim in einem Reaktor (1) zusammengeführt werden; wobei diese Rohstoffe zumindest aus Harnstoff, Wasser und einem Harnstoff-Formaldehyd-Konzentrat bestehen; wobei das Harnstoff-Formaldehyd-Konzentrat durch Kombination von Harnstoff, Formaldehyd und Wasser erzielt wird, und das erzielte Harnstoff-Formaldehyd-Konzentrat mit Hilfe einer ersten Leitung (7) in einen Vorratsbehälter (2, 20) eingeleitet wird; wobei das Harnstoff-Formaldehyd-Konzentrat aus dem Vorratsbehälter (2, 20) mit Hilfe einer zweiten Leitung in den Reaktor (1) eingeführt wird, **dadurch gekennzeichnet, dass** die Formaldehyd/Harnstoff-Molverhältnisse des Harnstoff-Formaldehyd-Konzentrats auf der Grundlage von in dem nahen Infrarotbereich (NIR) durchgeführten Messungen und mindestens einem Kalibrierungsmodell bestimmt werden; und dass diese in dem nahen Infrarotbereich durchgeführten Messungen an einem Harnstoff-Formaldehyd-Konzentrat an mindestens zwei Stellen durchgeführt werden, nämlich an einer ersten Stelle, die sich in der ersten Leitung (7) befindet, und an einer zweiten Stelle, die sich in dem Vorratsbehälter (2, 20) oder in der zweiten Leitung befindet; und wobei die Rohstoffe auf der Grundlage der oben genannten Formaldehyd/Harnstoff-Molverhältnisse des Harnstoff-Formaldehyd-Konzentrats dosiert in den Reaktor (1) eingebracht werden.

2. Verfahren nach Anspruch 1, wobei das Harnstoff-Formaldehyd-Konzentrat in einem Durchlaufproduktionsprozess hergestellt wird und dieser Durchlaufproduktionsprozess zumindest auf der Grundlage der in dem nahen Infrarotbereich an dem Harnstoff-Formaldehyd-Konzentrat durchgeführten Messungen, vorzugsweise zumindest auf der Grundlage der in dem nahen Infrarotbereich an dem Harnstoff-Formaldehyd-Konzentrat der ersten Stelle durchgeführten Messungen, gesteuert wird.

3. Verfahren nach Anspruch 1 oder 2, wobei die Rohstoffe auf der Grundlage der oben genannten Formaldehyd/Harnstoff-Molverhältnisse des Harnstoff-Formaldehyd-Konzentrats von der ersten Stelle und/oder der zweiten Stelle dosiert in den Reaktor (1) eingebracht werden.

4. Verfahren nach einem der vorhergehenden Ansprüche, **dadurch gekennzeichnet, dass** zumindest an der ersten und/oder zweiten Stelle, die in dem nahen Infrarotbereich an dem Harnstoff-Formaldehyd-Konzentrat durchgeführten Messungen online durchgeführt werden.

5. Verfahren nach Anspruch 4, wobei die in dem nahen Infrarotbereich an dem Harnstoff-Formaldehyd-Konzentrat der ersten und/oder zweiten Stelle durchgeführten Messungen teilweise auch offline durchgeführt werden; wobei das Kalibrierungsmodell für Online-Messungen in dem nahen Infrarotbereich mittels entsprechender in dem nahen Infrarotbereich durchgeführter Messungen, die offline durchgeführt wurden, optimiert wird.

6. Verfahren nach einem der vorhergehenden Ansprüche, **dadurch gekennzeichnet, dass** das Kalibrierungsmodell mindestens unter Verwendung von Messungen von Referenzproben in dem nahen Infrarotbereich formuliert wird; und wobei das Kalibrierungsmodell vorzugsweise auf der Grundlage von Messungen in dem nahen Infrarotbereich optimiert wird, die während der Herstellung des Harnstoff-Formaldehyd-Konzentrats durchgeführt wurden.

7. Verfahren nach einem der vorhergehenden Ansprüche, **dadurch gekennzeichnet, dass** mindestens zwei Kalibrierungsmodelle verwendet werden, um die Formaldehyd/Harnstoff-Molverhältnisse des Harnstoff-Formaldehyd-Konzentrats zu bestimmen, und zwar mindestens ein erstes Kalibrierungsmodell, das für Messungen verwendet wird, die in dem nahen Infrarotbereich an einem Harnstoff-Formaldehyd-Konzentrat der ersten Stelle durchgeführt werden, und ein zweites Kalibrierungsmodell, das für Messungen verwendet wird, die in dem nahen Infrarotbereich an einem Harnstoff-Formaldehyd-Konzentrat der zweiten Stelle durchgeführt werden.

8. Verfahren nach einem der vorhergehenden Ansprüche, wobei die Messungen in dem nahen Infrarotbereich an einem Harnstoff-Formaldehyd-Konzentrat mit Hilfe einer Sonde durchgeführt werden, durch die das Harnstoff-Formaldehyd-Konzentrat fließt, vorzugsweise durch die das Harnstoff-Formaldehyd-Konzentrat mit einer Fließgeschwindigkeit zwischen 0,05 m/s und 0,34 m/s fließt; und dass die mit der Sonde durchgeführten Messungen in dem nahen Infrarotbereich vorzugsweise Messungen darstellen, die in dem nahen Infrarotbereich mittels Transmission durchgeführt werden.

9. Verfahren nach einem der vorhergehenden Ansprüche, wobei die Rohstoffe zusätzlich einen oder mehrere Rohstoffe umfassen, die aus der Liste ausgewählt sind, die Folgendes umfasst: Melamin und Zusatzstoffe wie Essigsäure, Natriumhydroxid und Natriumacetat.

10. Verfahren nach einem der vorhergehenden Ansprüche, wobei die in dem nahen Infrarotbereich an dem Harnstoff-Formaldehyd-Konzentrat der zweiten Stelle durchgeführten Messungen mit den entsprechenden in dem nahen Infrarotbereich an dem Harnstoff-Formaldehyd-Konzentrat der ersten Stelle durchgeführten Messungen in Verbindung gebracht werden; wobei mindestens ein erstes Kalibrierungsmodell für die in dem nahen Infrarotbereich durchgeführten Messungen an dem Harnstoff-Formaldehyd-Konzentrat der ersten Stelle verwendet wird, und mindestens ein zweites Kalibrierungsmodell für die in dem nahen Infrarotbereich durchgeführten Messungen an dem Harnstoff-Formaldehyd-Konzentrat der zweiten Stelle verwendet wird; wobei mindestens eines der Kalibrierungsmodelle auf der Grundlage des Verbindens der in dem nahen Infrarotbereich durchgeführten Messungen optimiert wird.

11. Verfahren nach einem der vorhergehenden Ansprüche, wobei das Harnstoff-Formaldehyd-Konzentrat in einem Durchlaufproduktionsprozess hergestellt wird, der an die erste Leitung (7) angeschlossen ist; und wobei von mindestens zwei Vorratsbehältern (2, 20) Gebrauch gemacht wird; wobei der Reaktor (1) abwechselnd aus diesen mindestens zwei Vorratsbehältern (2, 20) befüllt wird; und wobei die erste Leitung (7) die beiden Vorratsbehälter (2, 20) abwechselnd nachspeist; wobei vorzugsweise einer der Vorratsbehälter (2, 20) immer nachgespeist wird, während der andere Vorratsbehälter (2, 20) in Richtung des Reaktors (1) entleert wird.

12. Verfahren nach einem der vorhergehenden Ansprüche, wobei die in dem nahen Infrarotbereich durchgeführten Messungen sich auf den erzielten Leim auf Harnstoff-Formaldehyd-Basis beziehen; wobei eine oder mehrere Eigenschaften dieses Leims auf der Grundlage dieser in dem nahen Infrarotbereich durchgeführten Messungen und eines jeweiligen Kalibrierungsmodells bestimmt werden.

13. Verfahren nach Anspruch 12, wobei die Herstellung des Substrats auf der Grundlage der oben genannten einen oder mehreren Eigenschaften des Leims auf Harnstoff-Formaldehyd-Basis erfolgt; wobei beispielsweise Vorbereitungsparameter wie die Presszeit, die Presstemperatur und die Menge des aufgetragenen Leims eingestellt werden und/oder Rohstoffe wie Formaldehydfänger und/oder eine zusätzliche Menge Harnstoff und/oder Härter wie Ammoniumnitrat während des Beschichtens der Holzpartikel mit dem Leim zugegeben werden.

## Revendications

1. Procédé destiné à la production d'une colle à base d'urée-formaldéhyde, dans lequel on combine des matières premières de cette colle dans un réacteur (1) ; dans lequel ces matières premières comprennent au minimum de l'urée, de l'eau et un concentré d'urée-formaldéhyde ; dans lequel on obtient le concentré d'urée-formaldéhyde en combinant de l'urée, du formaldéhyde et de l'eau, et on introduit dans un réservoir de stockage (2, 20) le concentré d'urée-formaldéhyde que l'on a obtenu, à l'aide d'une première ligne (7) ; dans lequel on introduit le concentré d'urée-formaldéhyde dans le réacteur (1) à partir du réservoir de stockage (2, 20) à l'aide d'une deuxième ligne, **caractérisé en ce que** l'on détermine les rapports molaires formaldéhyde/urée du concentré d'urée-formaldéhyde sur la base de mesures réalisées dans le proche infrarouge (NIR) et d'au moins un modèle d'étalonnage ; et **en ce que** ces mesures réalisées dans le proche infrarouge sont réalisées sur un concentré d'urée-formaldéhyde à au moins deux endroits, à savoir un premier endroit qui est situé dans la première ligne (7) et un deuxième endroit qui est situé dans le réservoir de stockage (2, 20) ou dans la deuxième ligne ; et dans lequel les matières premières sont introduites de manière dosée dans le réacteur (1) sur la base desdits rapports molaires formaldéhyde/urée du concentré d'urée-formaldéhyde.

2. Procédé selon la revendication 1, dans lequel on obtient le concentré d'urée-formaldéhyde dans un procédé de production en continu et ce procédé de production en continu est au moins géré sur la base desdites mesures réalisées dans le proche infrarouge du concentré d'urée-formaldéhyde, de préférence au moins sur la base desdites mesures réalisées dans le proche infrarouge du concentré d'urée-formaldéhyde du premier endroit.

3. Procédé selon la revendication 1 ou 2, dans lequel on introduit les matières premières de manière dosée dans le réacteur (1) sur la base desdits rapports molaires formaldéhyde/urée du concentré d'urée-formaldéhyde du premier endroit et/ou du deuxième endroit.

4. Procédé selon l'une quelconque des revendications précédentes, **caractérisé en ce qu'**au moins au premier et/ou au deuxième endroit, les mesures concernant le concentré d'urée-formaldéhyde dans le proche infrarouge sont réalisées en ligne.

5. Procédé selon la revendication 4, dans lequel les mesures concernant le concentré d'urée-formaldéhyde réalisées dans le proche infrarouge du premier et/ou du deuxième endroit sont également réalisées en partie hors ligne ; dans lequel on optimise le modèle d'étalonnage pour des mesures réalisées en ligne dans le proche infrarouge au moyen de mesures correspondantes réalisées dans le proche infrarouge qui ont été réalisées hors ligne.

6. Procédé selon l'une quelconque des revendications précédentes, **caractérisé en ce que** l'on formule au moins ledit modèle d'étalonnage en utilisant des mesures d'échantillons de référence réalisées dans le proche infrarouge ; et dans lequel on optimise de préférence le modèle d'étalonnage sur la base de mesures réalisées dans le proche infrarouge, qui ont été réalisées au cours de la préparation du concentré d'urée-formaldéhyde.

7. Procédé selon l'une quelconque des revendications précédentes, **caractérisé en ce que** l'on utilise au moins deux modèles d'étalonnage pour déterminer les rapports molaires formaldéhyde/urée du concentré d'urée-formaldéhyde, à savoir au moins un premier modèle d'étalonnage que l'on utilise pour des mesures réalisées dans le proche infrarouge sur un concentré d'urée-formaldéhyde du premier endroit et un deuxième modèle d'étalonnage que l'on utilise pour des mesures réalisées dans le proche infrarouge sur un concentré d'urée-formaldéhyde du deuxième endroit.

8. Procédé selon l'une quelconque des revendications précédentes, dans lequel on réalise les mesures dans le proche infrarouge sur un concentré d'urée-formaldéhyde à l'aide d'une sonde à travers laquelle s'écoule le concentré d'urée-formaldéhyde, de préférence à travers laquelle le concentré d'urée-formaldéhyde s'écoule à une vitesse d'écoulement entre 0,05 m/s et 0,34 m/s ; et en ce que les mesures réalisées dans le proche infrarouge à l'aide de la sonde représentent de préférence des mesures réalisées dans le proche infrarouge par le biais d'une transmission.

9. Procédé selon l'une quelconque des revendications précédentes, dans lequel les matières premières comprennent en outre une ou plusieurs matières premières choisies parmi la liste qui comprend : de la mélamine et des additifs tels que l'acide acétique, l'hydroxyde de sodium et l'acétate de sodium.

10. Procédé selon l'une quelconque des revendications précédentes, dans lequel les mesures réalisées dans le proche infrarouge concernant le concentré d'urée-formaldéhyde du deuxième endroit sont mises en liaison avec les mesures correspondantes réalisées dans le proche infrarouge concernant le concentré d'urée-formaldéhyde du premier endroit ; dans lequel on fait usage d'au moins un premier modèle d'étalonnage pour les mesures réalisées dans le proche infrarouge concernant le concentré d'urée-formaldéhyde du premier endroit, et on fait usage d'au moins un deuxième modèle d'étalonnage pour les mesures réalisées dans le proche infrarouge concernant concentré d'urée-formaldéhyde du deuxième endroit ; dans lequel on optimise au moins un desdits modèles d'étalonnage sur la base de ladite mise en liaison des mesures réalisées dans le proche infrarouge.

11. Procédé selon l'une quelconque des revendications précédentes, dans lequel on prépare le concentré d'urée-formaldéhyde dans un procédé de production en continu qui est raccordé à la première ligne (7) ; et dans lequel on fait usage d'au moins deux réservoirs de stockage (2, 20) ; dans lequel on remplit le réacteur (1) en alternance à partir de ces au moins deux réservoirs de stockage (2, 20) ; et dans lequel la première ligne (7) réalimente en alternance les deux réservoirs de stockage (2, 20) ; dans lequel, de préférence, un desdits réservoirs de stockage (2, 20) est toujours réalimenté, tandis que l'autre réservoir de stockage (2, 20) se vide en direction du réacteur (1).

12. Procédé selon l'une quelconque des revendications précédentes, dans lequel les mesures réalisées dans le proche infrarouge concernent la colle à base d'urée-formaldéhyde que l'on a obtenue ; dans lequel une ou plusieurs caractéristiques de cette colle sont déterminées sur la base de ces mesures réalisées dans le proche infrarouge et d'un modèle d'étalonnage respectif.

13. Procédé selon la revendication 12, dans lequel la préparation du substrat a lieu sur la bases desdites une ou plusieurs caractéristiques de la colle à base d'urée-formaldéhyde ; dans lequel, par exemple, on règle des paramètres de préparation, tels que la durée de compression, la température de compression la quantité de la colle que l'on applique et/ou on ajoute des matières premières supplémentaires, telles que des capteurs de formaldéhyde, et/ou une quantité supplémentaire d'urée et/ou des durcisseurs, tels que le nitrate d'ammonium, au cours de l'enduction des particules de bois avec la colle.
